# FASCICULE DE BREVET EUROPEEN

(11) **EP 3 558 468 B1**
(45) Date de publication et mention de la délivrance du brevet: **27.01.2021**
(21) Numéro de dépôt: 17825239.1
(22) Date de dépôt: 22.12.2017
(51) Int. Cl.: A61Q 19/08, A61K 8/98

(54) **COMPOSITION COSMETIQUE COMPRENANT DE LA GELEE ROYALE D'ABEILLE NOIRE D'OUESSANT**
KOSMETISCHE ZUSAMMENSETZUNG MIT GELEE ROYALE DER SCHWARZEN BIENE VON OUESSANT
COSMETIC COMPOSITION COMPRISING ROYAL JELLY OF THE OUESSANT BLACK BEE

(30) Priorité: 22.12.2016 FR 1663154
(43) Date de publication de la demande: 30.10.2019
(73) Titulaire: LVMH Recherche, 45800 Saint Jean de Braye (FR)
(72) Inventeur: KURFURST, Robin, 45800 Saint Jean de Braye (FR); SOBILO, Lauren, 45400 Chanteau (FR); JUAN, Milene, 45800 Saint Jean de Braye (FR); LEBLANC, Emmanuelle, 45560 Saint Denis en Val (FR); JEANNETON, Olivier, 45530 Vitry aux Loges (FR); ARCHAMBAULT, Jean-Christophe, 45130 Meung s/Loire (FR)
(74) Mandataire: Regimbeau
(86) Numéro de dépôt international: PCT/EP2017/084402
(87) Numéro de publication internationale: WO 2018/115448

(56) Documents cités:
- EP-A1- 1 438 964
- US-A1- 2009 068 128
- US-A1- 2011 268 812
- DATABASE GNPD [Online] MINTEL; 10 février 2016 (2016-02-10), "Rich Day Cream Firming, Wrinkle Minimizing, Radiance", XP002772208, Database accession no. 4329035
- DATABASE GNPD [Online] MINTEL; septembre 2015 (2015-09), "Gold Eyetech Eye Sculpt Serum", XP002772209, Database accession no. 3398269
- DATABASE GNPD [Online] MINTEL; novembre 2013 (2013-11), "Face Treatment Oil", XP002772210, Database accession no. 2238472
- DATABASE GNPD [Online] MINTEL; 9 février 2010 (2010-02-09), "Youth Serum", XP002772211, Database accession no. 1396935
- DATABASE GNPD [Online] MINTEL; 3 février 2001 (2001-02-03), "Ultimate Repair Cream", XP002772212, Database accession no. 3852597
- DATABASE GNPD [Online] MINTEL; avril 2016 (2016-04), "Extraordinary Oil-Cream", XP002772213, Database accession no. 3927391

## Description

La présente invention a pour objet une composition cosmétique favorisant la régénération et/ou la cicatrisation de la peau comprenant de la gelée royale d'abeille noire d'Ouessant (*Apis mellifera mellifera*) dans un milieu cosmétiquement acceptable.

La cicatrisation et la régénération tissulaire sont des processus complexes et intimement liés, mettant en jeu de nombreux types cellulaires, impliquant de multiples systèmes de régulation, une communication cellulaire complexe et la sécrétion d'un grand nombre de facteurs de transcription (*Martin, 1997*).

Le TGF-béta (TGF-B) est le principal déclencheur et régulateur des trois grandes étapes du processus de cicatrisation que sont l'inflammation, la prolifération cellulaire et le remodelage tissulaire. Par son spectre d'activité et ses actions en amont de ce processus, la voie du TGF-β est la voie biologique qui permet d'accélérer ou au contraire ralentir les mécanismes de cicatrisation.

Le TGF-β agit en se liant à ses récepteurs cellulaires formant un complexe de récepteurs de type I (TβR-I) et de type II (TβR-II) et via l'activation de la voie des smads, conduit ainsi à une régulation positive d'un ensemble de gènes. Ces gènes régulés par TGF-β sont impliqués dans la synthèse de protéines de la matrice extracellulaire, la prolifération et la motilité cellulaire, la régulation négative de l'expression d'enzymes protéolytiques ou encore la prolifération des fibroblastes dermiques.

La voie du TGF-β est altérée par le vieillissement intrinsèque et le photovieillissement conduisant ainsi à un amincissement cutané et à des retards de cicatrisation tels que ceux observés par exemple chez les personnes âgées. Une autre caractéristique de la peau âgée est la réduction de la synthèse du collagène qui conduit à un amincissement de la peau et à une fragilité accrue. Cependant, une action biologique qui viserait à augmenter la production ou la concentration de TGF-β pour atténuer les effets du vieillissement serait inefficace puisque c'est une altération des récepteurs au TGF-β qui est la cause de la perte d'activité du TGF-β au niveau cellulaire et tissulaire.

Par ailleurs, TIEG-1 (TGF beta early inducible gene 1) a été identifié en aval de la voie du TGF-β, comme un facteur clef de la régénération osseuse (Subramaniam M. et al. Mol. Cell. Biol., 2005, 1191-1199), de la cicatrisation (Taguchi M. et al., J. Musculoskelet. Res., 2008, 11, 63-69) mais aussi de l'organisation moléculaire des fibres de collagène et de la composition de la matrice collagénique (Gumez L. et al., J. Appl. Physiol., 2010, 108, 1706-1710).

Des études récentes rapportées par Subramaniam M. et al. (Biofactors, 2010, 36, 8-18) ont démontré l'implication du gène codant pour TIEG-1 dans la régénération osseuse. En effet, son expression est un élément de la réponse primaire des ostéoblastes (cellules synthétisant la partie non-minérale des os) au TGF-β. L'identification de la protéine TIEG-1, en aval de la voie du TGF-β, représente donc une cible moléculaire d'intérêt pour activer la voie de signalisation du TGF-β, en particulier lorsque les récepteurs au TGF-β sont altérés.

Dans ce contexte, les inventeurs ont montré de manière surprenante, que l'expression du gène TIEG-1 pouvait être spécifiquement augmentée sous l'effet d'une nouvelle composition cosmétique comprenant de la gelée royale d'abeille noire d'Ouessant (*Apis mellifera mellifera*). Les inventeurs ont également démontré que cette composition pouvait améliorer la régénération de la peau qui est altérée par les processus intrinsèque ou extrinsèque de vieillissement ou consécutivement à une agression, par exemple une agression physique. On parlera dans ce dernier cas de cicatrisation.

En particulier, les inventeurs ont mis en évidence que gelée royale d'abeille noire d'Ouessant permettait de stimuler l'expression de TIEG-1, et en particulier au sein d'une zone cicatricielle par rapport à une zone non-cicatricielle.

La gelée royale est l'un des produits issus de l'apiculture comme le miel, la propolis, le pollen, le venin d'abeille et la cire. Ces produits sont toutefois distincts les uns des autres, tant par leur mode de production au sein de la ruche que par leur composition et leurs propriétés.

Ainsi, le miel est produit par les abeilles qui butinent, qui collectent le nectar des fleurs et le fabriquent par des digestions et régurgitations successives. Le pH acide de l'estomac des abeilles ainsi que les activités enzymatiques de l'invertase, la diastase et l'amylase, donnent lieu à une solution aqueuse supersaturée, composée de 80% de sucres, principalement du fructose et du glucose, avec des quantités plus faibles de sucrose, maltose et autres sucres complexes.

Le miel et le pollen sont des substances qui constituent l'alimentation des abeilles pendant toute l'année, le premier apportant les sucres, le second les protéines. Le miel et le pollen peuvent être stockés pendant de longues périodes dans la ruche.

Les ouvrières sont une catégorie d'abeilles qui assurent de multiples tâches essentielles au maintien et à la survie de la colonie, la reine et les faux-bourdons assurant le reproduction de la colonie. Ce sont les ouvrières qui produisent de la gelée royale à partir de pollen et de nectar récoltés par les abeilles sur les fleurs, la gelée royale résultant de la sécrétion de leurs glandes salivaires mandibulaires et hypopharyngiennes.

La gelée royale constitue la nourriture de toutes les larves de la ruche, sans exception, pendant les trois premiers jours de leur vie, et la nourriture exclusive de la reine, pendant toute la vie de cette dernière. Par sa composition très complète la gelée royale apporte tous les éléments nutritifs nécessaires à la croissance des larves et à l'équilibre de la reine : une grande quantité d'eau (entre 60 et 70%), des sucres (entre 9 et 23%), des protéines (entre 10 et 18%) dont une grande partie d'acides aminés, et des lipides (entre 4 et 8%). Elle se présente sous la forme d'un colloïde acide, gélatineux, blanc-jaune, et comprend un acide gras spécifique : le 10-HDA (acide 10-hydroxy-2-décénoïque), identifié comme responsable d'une activité importante attachée aux stratégies de développement de la colonie.

La gelée royale présente une grande variabilité en fonction de la variétés des abeilles et de l'environnement dans lequel elles évoluent.

L'abeille noire d'Ouessant est une race pure d'un écotype local de l'Abeille Noire d'Europe occidentale (*Apis mellifera mellifera*). La situation géographique de l'île d'Ouessant, située à plus de 20 km de côtes finistériennes, constitue une barrière infranchissable ayant permis de préserver l'abeille noire d'Ouessant de toute hybridation avec d'autres espèces abeilles. Par ailleurs, l'île d'Ouessant est un écosystème qui a été protégé de tout temps et cela permet de garantir aux abeilles un environnement sain et diversifié ce qui confère aux abeilles et à leurs produits des caractéristiques uniques.

La présente invention a donc pour objet une composition cosmétique comprenant de la gelée royale d'abeille noire d'Ouessant dans un milieu cosmétiquement acceptable.

Si l'utilisation du miel d'abeilles noires d'Ouessant dans des compositions cosmétiques est connue depuis plusieurs années, il n'a jamais été envisagé d'utiliser la gelée royale d'abeilles noires d'Ouessant. Il s'agit en effet de deux produits distincts, aux compositions respectives distinctes et aux propriétés respectives distinctes.

De plus, contrairement à de la gelée royale « classique », ne provenant pas d'abeille noire d'Ouessant (*Apis mellifera mellifera*), la gelée royale d'abeille noire d'Ouessant présente l'avantage de stimuler l'expression de TIEG-1, notamment dans la zone cicatricielle, dans un modèle de cicatrisation *in vitro* par arrachage du tapis cellulaire et recolonisation.

Par « *cosmétiquement acceptable* » on entend que la composition convient à une utilisation topique, en contact avec la peau de mammifère et plus particulièrement la peau humaine.

Selon un mode de réalisation préféré, la composition cosmétique selon l'invention comprend de la gelée royale d'abeille noire d'Ouessant à une concentration comprise entre 0,001% et 1%, préférentiellement entre 0,005% et 0,5% et plus préférentiellement encore entre 0,05% et 0,2% en poids de la composition totale. Selon un autre objet de la présente invention, la composition cosmétique selon l'invention est plus particulièrement destinée à la régénération et/ou cicatrisation de la peau.

La composition de l'invention est en particulier destinée à des peaux présentant des signes de vieillissement chronologique ou de photovieillissement.

En effet, le vieillissement intrinsèque ou extrinsèque de la peau provoque un ralentissement du renouvellement cellulaire et une dégradation de la matrice extracellulaire de façon plus importante que sa néo-synthèse, ce qui indique une perte de l'homéostasie cutanée. Ceci se traduit notamment par un amincissement de la peau, un relâchement de la peau, ainsi que l'apparition de rides et ridules.

Selon un mode de réalisation préférée, la composition de l'invention est destinée à une application topique.

La composition cosmétique selon l'invention comprend, outre la gelée royale d'abeille noire d'Ouessant, un ou plusieurs excipients cosmétiques acceptable parmi ceux connus de l'homme du métier en vue d'obtenir une composition pour l'application topique sous forme de lait, de crème, de pommade, d'émulsion eau-dans huile, ou huile dans eau, de baume, de gel, de lotion, de sérum, de spray, préférentiellement de crème ou de sérum.

Selon la nature de la composition, on sélectionnera un ou plusieurs excipients cosmétiquement acceptables parmi des polymères, des agents tensioactifs, des agents de rhéologie, des parfums, des électrolytes, des ajusteurs de pH, des agents anti-oxydants, des conservateurs, des colorants, des nacres, des pigments et leurs mélanges.

Dans un mode de réalisation particulier, la composition cosmétique selon la présente invention peut comprendre en outre au moins un agent cosmétiquement actif bien connu de l'homme du métier choisis parmi des agents pour retarder ou ralentir l'apparition de signes du vieillissement cutané intrinsèque ou extrinsèque ; des agents ayant une activité dépigmentante ou une activité éclaircissante de la peau; des agents ayant une activité amincissante ; des agents ayant une activité hydratante ; des agents ayant une activité calmante, apaisante ou relaxante ; des agents stimulant la microcirculation cutanée pour améliorer l'éclat du teint, en particulier du visage ; des agents ayant une activité sébo-régulatrice pour le soin des peaux grasses ; des agents destinés à nettoyer ou purifier la peau ; des agents ayant une activité anti-radicalaire.

La composition cosmétique de l'invention peut ainsi comprendre une ou plusieurs autres substances pouvant être avantageusement choisies parmi :
- des molécules favorisant le renouvellement cellulaire telles que, le rétinol et/ou ses esters; des alpha ou beta hydroxy acides tels que les acides de fruits, notamment l'acide malique, l'acide glycolique ou l'acide citrique, l'acide salicylique ou ses esters, l'acide gentisique ou ses esters, en particulier le gentisate de tocopherol ;
- des molécules ou extraits stimulant la fermeté de la peau telles que des peptides stimulateurs de la synthèse de collagène, en particulier le collagène de type I, II, IV ou VII, un extrait de Centella asiatica, l'acide madécassique, l'acide asiatique, le madécassoside, un extraits d'avoine, un extrait de Berthollétia exscelsa, un hydrolyzat de protéines ou des peptides de soja, un extrait de Potentilla erecta, un extrait de Siegesbeckia orientalis, des ginsenosides ou notoginsenosides, un extrait d'écorce d'albizia julibrissin, un extrait de feuille de romarin riche en acide ursolique,

- des molécules ou extraits favorisant la synthèse d'acide hyaluronique ou de glycosaminoglycanes au niveau épidermique et dermique, tels qu'un extrait Essence vitale de Mamaku, un extrait de feuilles de Cyathea medullaris, un extrait d'Eriobotrya japonica ou des petits fragments d'acide hyaluronique de bas poids moléculaires ou bien encore un extrait d'Adenum obesum ;
- des molécules ou extraits régulant la différentiation de l'épiderme tels que l'ecdysterone, la turkesterone, des dérivés de calcium, des précurseurs de vitamine D ;
- l'adénosine, la carnitine ou ses dérivés en particulier l'acetylcarnitine, le rétinol des esters cosmétiquement acceptable, en particulier le propionate ou un palmitate de retinol ;
- des inhibiteurs de métalloprotéinases (MMP), en particulier des inhibiteurs des MMP 1, 2, 9 tel qu'un extrait de Ruscus asculeatus, des peptides de soja ou des des extraits végétaux des flavonoïdes en contenants ;
- des inhibiteurs d'élastase tels que des extraits végétaux d'Aspergillus fumigatus, de Momordica charantia, de Cucurbita maxima ;
- un peptide analogue de l'élastine, avantageusement estérifié, tel que celui formé par la séquence palmitoyl-Val-Gly-Val-Ala-Pro-Gly commercialisé sous la dénomination commerciale BIOPEPTIDE EL par la société SEDERMA ;
- des substances capables de stimuler la synthèse de dermatopontine, telles qu'un extrait d'ambre ;
- des molécules ou extraits de plantes astringents resserrant les pores tels qu'un extrait d'Hamamélis ; du gluconate de zinc
- des filtres protégeant des radiations UVA et UVB, tels que la benzophenone 4-butyl methoxydibenzoylmethane, l'éthylhexyl méthoxycinnamate, l'octocrylène, l'éthylhexyl salicylate, l'acide sulfonique phenylbenzymidazole, l'homosalate, seuls ou en association avec des oxydes de titane ;
- des molécules ou extraits végétaux agissant sur la pigmentation telles que l'acide kojique, les extraits de racine de réglisse ou de mûrier, l'arbutine, le pantothenosulfonate de calcium, la boldine, la diacetylboldine, la vitamine C, ou un de ses dérivés, tels que les glycosides, des extraits de lys en particulier de bulbe.
- des molécules ou extraits antiradicalaires ou anti-inflammatoires tels qu'un extrait d'Artemisia capillaris, un extrait de Sanguisorba officinalis, le resvératrol et ses dérivés, le cucurma, la cucurmine ou la tetrahydrocucurmine, des polyphénols extraits de pépins de raisin, de la vitamine E et ses dérivés, en particulier ses dérivés phosphate, l'ergothionéine ou ses dérivés, l'idébenone, ;
- un extrait d'orchidée telle qu'une orchidée appartenant au genre Brassocattleya, par exemple un extrait de l'orchidée Brassocattleya marcella, ou au genre Vanda, par exemple un extrait d'une orchidée parmi Vanda coerulea, Vanda teres ou encore Vanda denisoniana ;
- des agents hydratants comme le glycérol, la trimethyl glycine ou des polyols naturels, des céramides naturels ou de synthèse, des eaux de sources ou minérales.

Avantageusement, la composition selon l'invention peut comprendre en outre un miel ou un extrait de miel. Le miel étant préférentiellement un miel unifloral ou polyfloral.

De préférence, le miel utilisé dans la composition selon l'invention peut être un miel de trèfle (Trifolium repens), un miel d'Ouessant, un miel d'euphorbe *(Euphorbia echinus)* ou un miel de corse

Sous quelle que forme que ce soit, la composition cosmétique de l'invention est appliquée sur une partie de la peau du corps, en particulier le visage, le cou, le décolleté et/ou les mains.

Selon un autre aspect, la présente invention concerne un procédé de traitement cosmétique pour stimuler la régénération de la peau et/ou la cicatrisation cutanée, caractérisé en ce qu'on applique sur la peau une quantité suffisante d'une composition cosmétique comprenant de la gelée royale d'abeille noire d'Ouessant, à titre d'agent cosmétiquement actif.

Les figures et exemples ci-après sont donnés à titre illustratif et ne sont pas limitatifs.

### FIGURES

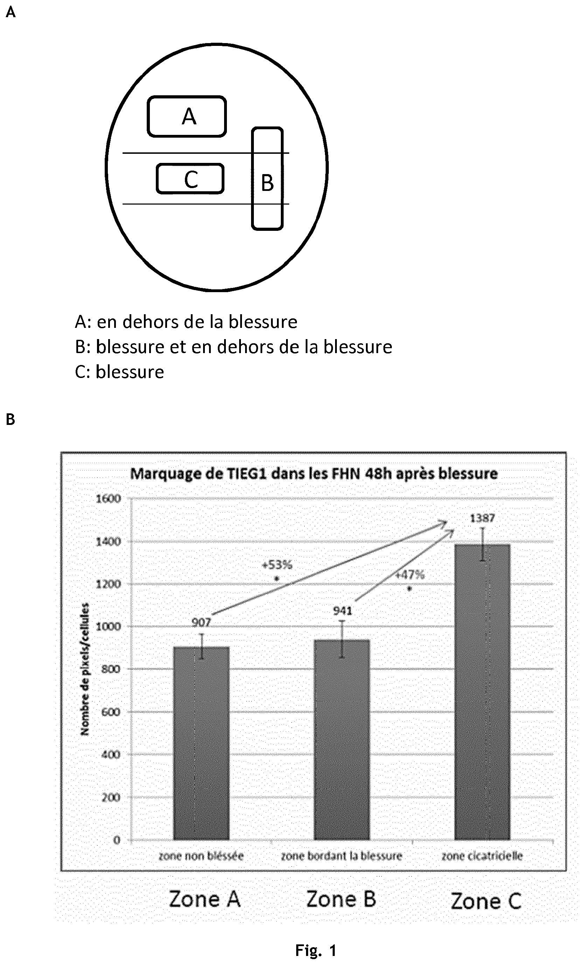

Figure 1 : Expression protéique de TIEG-1 dans un modèle de cicatrisation *in vitro* dans des FHN.
   A. Schéma des zones d'intérêts où un signal immunofluorescent est quantifié dans un modèle de cicatrisation. Zone A : éloignée de la zone blessée ; zone B : zone mixte comprenant la zone limitrophe de la blessure et une zone témoin sur le tapis cellulaire; zone C : zone de blessure correspondant à la zone raclée par une pointe de cône ; B. Suivi de l'expression protéique de TIEG-1 dans les fibroblastes 48h après blessure.
Figure 2 : Effet de la gelée royale d'abeille noire d'Ouessant sur le niveau protéique de TIEG1 dans des FHN en culture au niveau de zones blessée (ou zone cicatricielle) et non-blessé.
Figure 3 : Effet de la gelée royale d'abeille noire d'Ouessant et de la gelée royale d'abeille jaune rayée noire (abeille Buckfast) sur la synthèse du Collagène de type V dans des FHN en culture.
Figure 4 : Effet de la gelée royale d'abeille noire d'Ouessant et de la gelée royale d'abeille jaune rayée noire (abeille Buckfast) sur la synthèse d'élastine dans des FHN en culture.

### EXEMPLES

### Exemple 1 : Expression de TIEG-1 dans un modèle de cicatrisation in vitro.

Il est possible d'obtenir par culture cellulaire, une surface couverte uniformément de cellules. Ce tapis peut être blessé en effectuant une déchirure par utilisation de la pointe d'un cône qui va racler le tapis cellulaire.

Sur ce modèle, on distingue 3 zones différentes : la zone de blessure correspondant à la zone raclée par la pointe de cône (zone C), une zone mixte c'est-à-dire la zone comprenant la zone limitrophe de la blessure (zone B) et une zone témoin sur le tapis cellulaire, éloignée de la zone blessée (Zone A) (Figure 1, A). L'expression de TIEG-1 a été révélée par immuno-marquage et quantifiée par analyse d'image dans les fibroblastes présents dans ces trois zones quand le processus de cicatrisation est amorcé.

Cette étude démontre que TIEG-1 est surexprimé significativement dans la zone de blessure C de 47% par rapport à la zone mixte B et de 53% par rapport à la zone témoin A (Figure 1, B).

Ceci indique que TIEG-1 est un acteur majeur du mécanisme de la mobilité cellulaire notamment fibroblastique, mécanisme majeur du processus de cicatrisation.

### Exemple 2 : Effet de la gelée royale d'abeille noire d'Ouessant sur l'expression protéique de TIEG 1 dans des fibroblastes humains normaux (FHN) en culture soumis à un test de cicatrisation

L'expression de TIEG 1 est évalué sous l'effet d'un traitement par de la gelée royale d'abeille noire d'Ouessant (0,01%) sur un modèle de cicatrisation *in vitro* de FHN en culture.

### 2.1. Traitement des cellules

Des FHN provenant d'une plastie abdominale d'une femme caucasienne de 37 ans sont ensemencés à raison de 80 000 cellules par boite de pétri 35 ibidi treat dans du milieu DMEM faible en glucose implémenté de 10% de SVF et d'un mélange d'antibiotiques (Pénicilline/Streptomycine) et maintenues en culture pendant 48 heures, puis le milieu est changé et remplacé par du milieu déplété en SVF.

Après 24 heures de culture sans SVF, les FHN sont traités avec la gelée royale d'abeille noire d'Ouessant à la dose de 100 µg/ml.

Après 15 heures de traitement, une blessure sur le tapis de FHN de chaque boîte de Pétri est réalisée à l'aide d'un cône, en forme de croix.

24h après la blessure, le milieu de culture est éliminé et l'immunomarquage de TIEG1 est réalisé.

Les cellules sont fixées à la formaline puis perméabilisées au triton (0,1%) pendant 10 minutes. Elles sont ensuite saturées avec du PBS/BSA 1% (« Phosphate Buffer Salin », « Bovin Serum Albumin ») pendant 30 minutes. L'anticorps primaire (anti-KLF10 de lapin; Abcam) est dilué au 1/200^{ème} dans du tampon PBS-BSA 1% et incubé sur les cellules à température ambiante pendant 1 heure. Après rinçage par du PBS, l'anticorps secondaire (« Alexa Fluor® 568 Goat Anti-Rabbit IgG ») est dilué au 1/200^{ème} dans du tampon PBS-BSA 1% et déposé à température ambiante pendant 1 heure à l'obscurité. Un marquage des noyaux au DAPI (dilué au 1/100^{ème}) ainsi que des filaments d'actine à la phalloïdine 488 (diluée au 1/200^{ème}) est réalisé en parallèle de l'incubation de l'anticorps secondaire.

Après rinçage au PBS, quelques gouttes de milieu de montage aqueux sont ajoutées. Les boites sont enfin stockées à 4°C en attente d'acquisition d'image au microscope à fluorescence.

### 2.2. Acquisition et analyse des images au microscope confocal

Les images sont réalisées avec un microscope confocal Leica SP5 II à l'objectif à air x20 à la résolution 1024 x 1024 pixels. Trois images sont faites en zone non blessée et trois images ou plus en zone blessée, avec les mêmes paramètres d'acquisition. Les images sont faites après excitation des fluorochromes par un laser spécifique : 1. laser à Argon (488 nm), 2. diode laser (405 nm) et 3. laser hélium-néon » (633 nm).

Une fois les acquisitions réalisées, les images sont analysées une par une grâce au logiciel Leica QWin, afin d'en obtenir une description quantitative.

Un programme d'analyse d'image permet une détection spécifique des noyaux et du marquage TIEG-1. La détection du marquage de TIEG-1 est réalisée dans le compartiment cytoplasmique et dans le noyau des cellules. Pour chaque condition, la quantification de TIEG-1 est mesurée dans la zone cicatricielle et dans la zone non blessée. Les valeurs de cette quantification sont systématiquement pondérées par le nombre de noyaux ou la surface cellulaire.

### 2.3. Résultats

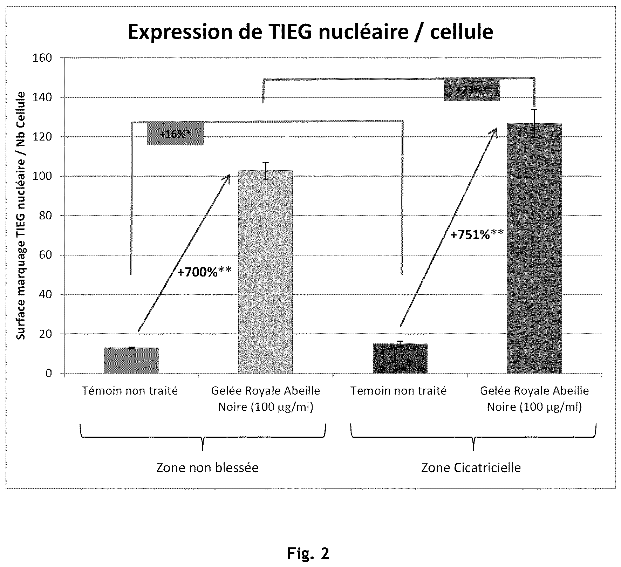

Les résultats (Figure 2) sont regroupés dans le tableau 1 ci-dessous pour les zones non blessée et blessée (zone cicatricielle).

**Tableau 1 : Expression nucléaire de TIEG1 dans la zone non blessée et dans la zone cicatricielle traitée avec de la gelée royale d'abeille noire d'Ouessant ou non traité.**

| | Conditions de traitement | Expression TIEG Noyaux/cellule | |
|---|---|---|---|
| | | Moyenne | Ecart type |
| Zone non blessée | Témoin non traité | 12,9 | 0,6 |
| | Gelée royale d'abeille noire d'Ouessant (100µg/ml) | 102,7 | 6,5 |
| Zone cicatricielle | Témoin non traité | 14,9 | 2,1 |
| | Gelée royale d'abeille noire d'Ouessant (100µg/ml) | 126,8 | 12,5 |

La gelée royale d'abeille noire d'Ouessant induit une augmentation significative de l'expression nucléaire de TIEG1 par rapport au témoin non traité de +700% dans la zone non blessée et de + 751% dans la zone cicatricielle (Figure 2).

Par ailleurs on note que le traitement par la gelée royale d'abeille noire d'Ouessant augmente de 23% l'expression de TIEG1 dans le compartiment nucléaire des cellules de la zone cicatricielle par rapport à l'expression de TIEG-1 après traitement des cellules de la zone non blessée, contre 16% pour le témoin non traité.

Ainsi le traitement par la gelée royale d'abeille noire d'Ouessant agit plus efficacement sur une zone cicatricielle, ce qui la rend particulièrement intéressante pour une utilisation en tant qu'agent anti-âge dans des compositions de soin cosmétique de la peau visant à prévenir ou ralentir l'apparition de signes du vieillissement intrinsèque ou extrinsèque de la peau.

### Exemple 3 : Etude de l'effet de gelée royale d'abeille noire d'Ouessant et de gelée royale d'abeille jaune rayée noire (abeille Buckfast) sur l'expression du collagène V et de l'élastine dans des fibroblastes humains normaux (FHN) en culture

### 3.1. Traitements des cellules

Les FHN sont ensemencés dans des flacons T75 (flasque de 75 cm2, BD Biosciences) dans du milieu DMEM (Dulbecco's modified Eagle's medium) supplémenté par 10% de sérum de veau fœtal

Les FHN sont trypsinés à confluence par une solution à 0,05% trypsin-EDTA, et neutralisés avec un milieu contenant du sérum. Puis ils sont ensemencés dans 2 plaques 24 puits à raison de 38 000 cellules par puits.

Les FHN sont ensuite traités (ou non) avec la gelée royale d'abeille noire d'Ouessant ou d'abeille jaune rayée noire (abeille Buckfast) testées aux concentrations de 100 µg/ml pendant 6 jours. L'abeille jaune rayée noire est issue du croisement des sous-espèces d'abeilles *Apis mellifera mellifera* et *Apis mellifera ligustica.* Le traitement est réalisé en double pour chaque condition et renouvelé à 3 reprises pendant ces 6 jours. Pour les conditions témoin NT (non traité), le milieu est remplacé par du milieu neuf.

Après 6 jours de traitement, les cellules sont rincées deux fois avec un tampon phosphate, puis fixées à la formaline (Solution à 10%) pendant 10 minutes. Après 2 rinçages au PBS, les membranes des cellules sont perméabilisées avec une solution de PBS/Triton X-100 à 0,1% (Sigma), puis rincées 2 fois au tampon phosphate.

Les cellules sont recouvertes d'une solution de sérum albumine bovin à 1% dans du tampon phosphate, pendant 30 minutes et à température ambiante.

La solution de PBS/BSA est ensuite remplacée par une solution d'anticorps primaire correspondant à chaque protéine marquée (voir tableau 2 ci-dessous) diluée au 1/100^{ème} dans du PBS/BSA 1%.

**Tableau 2 : Récapitulatif des anticorps utilisés**

| | **Anticorps Primaire** | **Anticorps Secondaire** |
|---|---|---|
| **Collagène V** | Novotec 20511, Rabbit au 1/500^{ème} | Alexa fluor 568 Goat Anti Rabbit (Molecular Probes) au 1/200^{ème} |
| **Elastine** | Novotec 25011, Rabbit au 1/200^{ème} | |

Les plaques sont incubées 2 heures à température ambiante.

Les cellules sont ensuite rincées avec du tampon phosphate et recouvertes d'une solution d'anticorps secondaire selon l'anticorps primaire à cibler (Tableau 2) et de DAPI (4',6'-diamidino-2-phenylindole, dihydrochloride) dilués respectivement au 1/200^{ème} et au 1/100^{ème} dans une solution de sérum albumine bovin à 1% dans du tampon phosphate1%. Les plaques sont conservées une heure à l'obscurité et à température ambiante.

La solution d'anticorps secondaires est ensuite aspirée et les cellules rincées au PBS, puis à l'eau distillée. Quelques gouttes de milieu de montage (Aqua-Mount, Lab Vision Thermo Scientific) sont déposées dans chaque puits.

### 3.2. Acquisition et analyse des images par criblage à haut contenu (High-Content Screening (HCS))

Les plaques sont scannées à l'« ArrayScan XTi » (Thermo Cellomics).

### Conditions d'acquisition :

Détection :
- DAPI : filtre XF53_386_23
- Alexa Fluor 568 : filtre XF53_572_15
- Résolution : 1104 x 1104
- Objectif : 10x sec
- Nombre d'images : 49 par puits (soit 2 x 49 = 98 par condition)

Les images sont analysées à l'aide d'un logiciel d'analyse d'image (« Spot detector »), qui détecte le marquage rouge de la protéine ciblée, correspondant à l'expression de celle-ci. La surface de la zone de mesure correspond à la totalité de la surface de l'image. Le nombre de cellules est déterminé par le comptage des noyaux par détection du marquage bleu.

### 3.3. Résultats

### a. Expression du collagène de type V

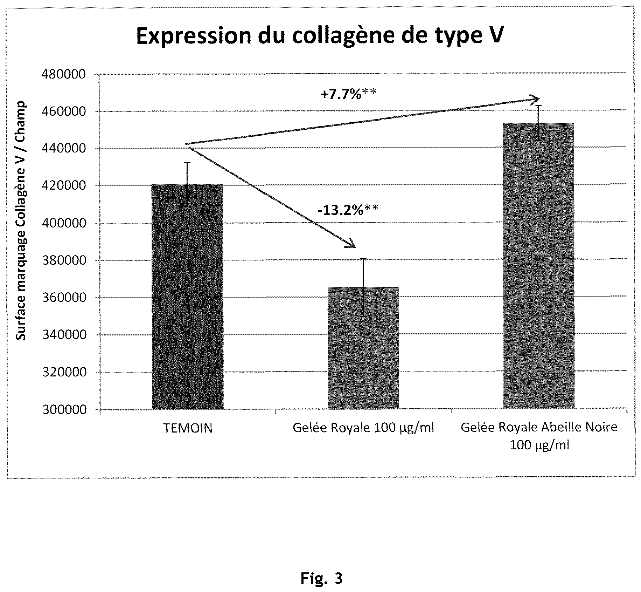

La gelée royale d'abeille noire d'Ouessant augmente significativement la synthèse du collagène de type V sur des FHN par rapport au témoin non traité (Figure 3).

La gelée royale d'abeille noire d'Ouessant a également un effet significativement supérieur à celui observé pour la gelée royale d'abeille jaune rayée noire (abeille Buckfast) (Figure 3).

### b. Expression de l'élastine

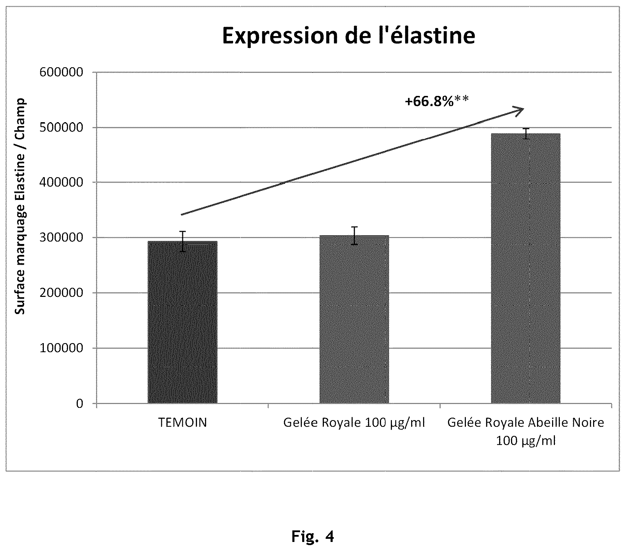

La gelée royale d'abeille noire d'Ouessant augmente très significativement (+66.8%) la synthèse d'élastine sur des FHN (Figure 4).

Les résultats obtenus sur différentes cibles impliquées dans la synthèse de la matrice extracellulaire dans des FHN montre que la gelée royale d'abeille noire d'Ouessant à un effet activateur sur l'expression du collagène de type V et de l'élastine, qui est significativement supérieur à celui mesuré pour la gelée royale d'abeille jaune rayée noire (abeille Buckfast). Aucun effet particulier sur l'expression du collagène de type V et de l'élastine n'est observé dans les FHN traité avec la gelée royale d'abeille jaune rayée noire.

Au contraire, la gelée royale d'abeille jaune rayée noire a montré qu'elle diminuait significativement (-13,2%) la synthèse du collagène de type V sur des FHN par rapport au témoin non traité.

### Exemple 4 : Crème de jour SPF 15

La composition ci-dessous est une émulsion huile-dans-eau.

La gelée royale d'abeille noire d'Ouessant est identifiée par une teneur élevée en acide 10-hydroxy-2-decenoïque par rapport à la teneur en ce composé dans une gelée d'abeille noire rayée jaune (Buckfast).

Les pourcentages sont exprimés en poids par rapport à la composition finale :

| | |
|---|---|
| Gelée royale d'abeille noire d'Ouessant | 0,01 |
| Octocrylen | 2,0 |
| Méthoxycinnate d'octyle | 7,5 |
| Ethyl 2 hexyle palmitate | 4,0 |
| Phenyl trimethicone | 0,5 |
| Glyceryl monostearate | 0,2 |
| Cetearyl alcohol/dicetylphopshate/ceteth-10 phosphate | 2,0 |
| Glyceryl stearate/ PEG-100 stearate | 2,0 |
| Alcool cétylique | 2,0 |
| Cire d'abeille Polyglyceryl-3 | 0,4 |
| Benzophenone-3 | 1,5 |
| Caprylic/caric triglyceride | 3,5 |
| Butylène glycol dicaprylate/dicaprate | 2,5 |
| C12-15 alkyl benzoate/ titanium dioxide/polyhydroxystearic acide / stéarate d'aluminum / alumina | 2,5 |
| Sodium polyacrylate | 0,6 |
| Butylène glycol | 2,0 |
| Diglycérine | 1,0 |
| Glycérol | 3,5 |
| Sodium hyaluronate | < 0,1 |
| Gomme xanthane | 0,2 |
| Ammonium acryloyldiméthyltaurate/VP copolymère | 0,4 |
| Acide citrique | < 0,1 |
| Citrate trisodique | 0,1 |
| Succinate octénylique d'**amidon** aluminique | 1,5 |
| Solution d'extrait de feuilles de romarin | 2,0 |
| Solution d'extrait d'écorce d'arbre à soie (PRODIZIA®) | 2,0 |
| Tocopheryl acetate | 0,2 |
| Adjuvants (parfums, alcalinisant, conservateurs) | qs |
| Eau purifiée | qsp 100 |

La gelée royale d'abeille noire d'Ouesssant est une pâte qui est mise en solution dans la phase continue aqueuse de la crème.

Chaque phase est préparée séparément puis la phase grasse est ajoutée sous agitation dans la phase aqueuse de façon à obtenir une dispersion homogène des gouttelettes de phase grasse dans la phase continue.

La crème a une texture particulièrement agréable à l'application. Elle est appliquée au réveil, sur le visage, par un massage léger, en insistant sur les zones présentant des signes de vieillissement tels que des rides ou ridules ou des zones présentant un relâchement de la peau.

### REFERENCES BIBLIOGRAPHIQUES

Biofactors, 2010, 36, 8-18*)*
Gumez L. et al., J. Appl. Physiol., 2010, 108, 1706-1710
Paul Martin, Wound Healing-Aiming for Perfect Skin Régénération, Science, 1997, 276, 5309, pp. 75-81*.*
Snyder L.R. Journal Of Chromatography, vol. 92, 1974, pages 223 - 230
Subramaniam M. et al. Mol. Cell. Biol., 2005, 1191-1199
Subramaniam M. et al., Biofactors, 2010, 36, 8-18
Taguchi M. et al., J. Musculoskelet. Res., 2008, 11, 63-69

## Revendications

1. Composition cosmétique **caractérisée en ce qu'**elle comprend de la gelée Royale d'abeille noire d'Ouessant dans un milieu cosmétiquement acceptable.

2. Composition cosmétique selon la revendication 1, **caractérisée en ce qu'**elle comprend ladite gelée royale à une concentration comprise 0,001% et 1%, préférentiellement 0,005% et 0,5% et plus préférentiellement encore entre 0,05% et 0,2% en poids de la composition totale.

3. Composition cosmétique selon la revendication 1 ou 2, pour son utilisation topique destinée à stimuler la régénération de la peau et/ou la cicatrisation.

4. Composition cosmétique selon l'une quelconque des revendications 1 à 3, **caractérisée en ce que** ladite composition comprend un ou plusieurs excipients cosmétiquement acceptables.

5. Composition cosmétique selon la revendication 4, **caractérisée en ce que** ledit excipient cosmétiquement acceptable est sélectionné parmi des polymères, des agents tensioactifs, des agents de rhéologie, des parfums, des électrolytes, des ajusteurs de pH, des agents anti-oxydants, des conservateurs, des colorants, des nacres, des pigments et leurs mélanges.

6. Composition cosmétique selon l'une quelconque des revendications 1 à 5, en ce qu'elle est sous forme émulsions huile-dans-eau ou eau-dans-l'huile, crème, huile, lait, lotions, sérum, préférentiellement sous forme de crème ou de sérum.

7. Composition cosmétique selon l'une quelconque des revendications 1 à 6, en ce qu'elle comprendre en outre un miel unifloral ou polyfloral ou un extrait de miel, préférentiellement un miel de trèfle (*Trifolium repens*) ou d'euphorbe.

## Patentansprüche

1. Kosmetische Zusammensetzung, **dadurch gekennzeichnet, dass** sie Gelee royale der Biene Abeille noire d'Ouessant in einem kosmetisch akzeptablen Medium umfasst.

2. Kosmetische Zusammensetzung nach Anspruch 1, **dadurch gekennzeichnet, dass** sie das Gelee royale in einer Konzentration umfasst, die zwischen 0,001% und 1%, vorzugsweise 0,005% und 0,5% und noch vorzugsweiser zwischen 0,05% und 0,2 Gew.-% der Gesamtzusammensetzung liegt.

3. Kosmetische Zusammensetzung nach Anspruch 1 oder 2 für ihre topische Verwendung, die dazu bestimmt ist, die Regeneration der Haut und/oder die Heilung zu stimulieren.

4. Kosmetische Zusammensetzung nach einem der Ansprüche 1 bis 3, **dadurch gekennzeichnet, dass** die Zusammensetzung einen oder mehrere kosmetisch akzeptable Hilfsstoffe umfasst.

5. Kosmetische Zusammensetzung nach Anspruch 4, **dadurch gekennzeichnet, dass** der kosmetisch akzeptable Hilfsstoff aus Polymeren, Tensiden, rheologischen Mitteln, Düften, Elektrolyten, pH-Einstellern, Antioxidantien, Konservierungsstoffen, Farbstoffen, Perlmutt, Pigmenten und ihren Gemischen ausgewählt ist.

6. Kosmetische Zusammensetzung nach einem der Ansprüche 1 bis 5, dadurch, dass sie in Form von Öl-in-Wasser- oder Wasser-in-Öl-Emulsionen, Creme, Öl, Milch, Lotionen, Serum, vorzugsweise in Form von Creme oder von Serum, vorliegt.

7. Kosmetische Zusammensetzung nach einem der Ansprüche 1 bis 6, dass sie ferner einen unifloralen oder polyfloralen Honig oder einen Honigextrakt, vorzugsweise einen Klee- (*Trifolium repens*) oder Euphorbia-Honig umfasst.

## Claims

1. Cosmetic composition **characterized in that** it comprises royal jelly of the Ouessant black bee in a cosmetically acceptable medium.

2. Cosmetic composition according to claim 1, **characterized in that** it comprises said royal jelly at a concentration comprised between 0.001% and 1%, preferably between 0.005% and 0.5%, and more preferably between 0.05% and 0.2% by weight of the total composition.

3. Cosmetic composition according to claim 1 or 2, for topical use to stimulate skin regeneration and/or healing.

4. Cosmetic composition according to any one of claims 1 to 3, **characterized in that** said composition comprises one or more cosmetically acceptable excipients.

5. Cosmetic composition according to claim 4, **characterized in that** said cosmetically acceptable excipient is selected from polymers, surfactants, rheology agents, fragrances, electrolytes, pH adjusters, antioxidants, preservatives, dyes, mother-of-pearl, pigments and mixtures thereof.

6. Cosmetic composition according to any one of claims 1 to 5, in that it is in the form of oil-in-water or water-in-oil emulsions, cream, oil, milk, lotions, serum, preferably in the form of cream or serum.

7. Cosmetic composition according to any one of claims 1 to 6, in that it further comprises unifloral or polyfloral honey or honey extract, preferably clover (*Trifolium repens*) or euphorbia honey.
